# EUROPEAN PATENT APPLICATION

(11) **EP 3 679 959 A1**
(43) Date of publication of application: **15.07.2020**
(21) Application number: 19158448.1
(22) Date of filing: 21.02.2019
(51) Int. Cl.: A61M 1/00, A61M 1/06

(54) **BREAST PUMP**

(30) Priority: 11.01.2019 NZ 19749888
(71) Applicant: Think Green Ltd., Orakei, Auckland 1071 (NZ)
(72) Inventor: Zhang, Shu Ting, 0632 Auckland (NZ)
(74) Representative: Studio Torta S.p.A.

(57) **Abstract**

A breast pump comprising: a container comprising a deformable body and a lid, the container further comprising an inlet and a vent, wherein the vent is provided with a vent valve which is configured to allow air flow out of the container through the vent but not into the container through the vent; a nipple engaging means for receiving milk expressed from a user's breast; and a flexible conduit fluidly connecting the nipple engaging means and the inlet of the deformable container. In use, milk expressed from the breast flows from the nipple engaging means through the flexible conduit to the container.

## Description

### Field of Invention

The present invention relates to a breast pump, and in particular, but not exclusively, to a breast pump having a container which is remote from a nipple engaging means.

### Background to the Invention

Many mothers prefer to breast feed instead of using powdered milk formulas for feeding babies. The American Academy of Pediatrics recommends exclusive breastfeeding for the first six months of a baby's life and breastfeeding with the introduction of foods for at least the first year of a baby's life to ensure the short and long-term health of the baby. However, there are situations where breastfeeding may not be possible or practical, for instance if the mother is unwell, working, travelling, or running errands. Breastfeeding may also be difficult if the baby is not latching well or if the mother has undergone breast surgery and is unable to be stimulated by the suckling reflex. In these cases, it is still possible to provide the baby with breast milk through the use of breast pumps. Breast pumps can also be used to catch letdown on the lateral breast while breastfeeding, to express milk in the case of oversupply and to relieve engorged, swollen breasts.

Breast pumps are devices which allow mothers to express breast milk that can then be stored and used at a later time. There are various different types of breast pumps including manual and electric pumps.

Most breast pumps of the prior art comprise a breast engaging portion, an electric pump to create suction on the nipple of the breast, and a reservoir for storing the expressed milk.

The use of electric pumps can be problematic. Electric pumps are often noisy, which may mean the pump cannot be used in some situations. Furthermore, the pump requires a power source. Often the power source is a battery, which may weigh anywhere between 500g and 1.8kg. Electric pumps can also contain a large number of parts, which may create significant work for users because each part that comes into contact with the breast milk may need to be washed after every use. Since most breast pumps require the entire apparatus to the held by the user while the milk is being expressed, this can be tiring. The use of the battery also limits the amount of time the pump can be used for between charges, and the performance of the battery degrades with repeated charging cycles. Electric pumps may also need to be pressed firmly to the breast during operation to prevent leakage. This can put extra pressure and weight on the breast during use.

Even breast pumps which do not use electricity may have a number of limitations. Typically, the breast engaging portion, pump and reservoir are all closely coupled and must all be located near the user's breast when in use. The combined bulk of the components means that the pump cannot be used underneath most types of clothes, meaning the pump is typically only used when the user has a high degree of privacy. Because of this, many existing breast pumps cannot be used discretely in public.

A further problem with some designs of breast pump is backflow of the milk when the pump is turned off. This may be messy and may result in wasted milk.

### Object of the Invention

It is an object of the invention to provide a breast pump which will overcome or ameliorate one or more problems with the breast pumps of the prior art.

Alternatively, it is an object of the invention to provide a breast pump that allows mothers to express milk while performing normal everyday activities, giving them freedom.

Alternatively, it is an object of the invention to at least provide the public with a useful choice.

### Summary of the Invention

Preferred aspects of the invention are set forth in the appended claims. Particular embodiments are described below in non-limiting terms.

According to a first embodiment of the invention, there is provided a breast pump comprising:
- a container comprising a deformable body and a lid, the container further comprising an inlet and a vent, wherein the vent is provided with a vent valve which is configured to allow air flow out of the container through the vent but not into the container through the vent;
- a nipple engaging means for receiving milk expressed from a user's breast; and
- a flexible conduit fluidly connecting the nipple engaging means and the inlet of the deformable container,
wherein, in use, milk expressed from the breast flows from the nipple engaging means through the conduit to the container.

Preferably the flexible conduit is at least 500mm long, more preferably at least 800mm long.

Preferably the flexible conduit comprises a silicone tube.

Preferably the flexible conduit has an outside diameter of no more than 10mm, preferably no more than 8mm and more preferably no more than 6mm.

Preferably, the conduit connecting portion is configured to direct the conduit away from the nipple engaging means in a direction such that, in use, at least an end portion of the conduit connected to the conduit connecting portion lies substantially alongside the user's body.

Preferably, in use, the nipple engaging means protrudes from a surface of the user's breast by a distance of 35mm or less.

Preferably, the nipple engaging means is resiliently deformable.

Preferably, the breast pump further comprises an inlet valve between the nipple engaging means and an interior of the container, wherein the inlet valve allows fluid to flow from the nipple engaging means towards the interior of the container, but not from the interior of the container towards the nipple engaging means.

Preferably the inlet valve is connected to or integral with the container.

Preferably one or both of the inlet valve and the vent valve comprises a duckbill valve.

Preferably the container comprises a conduit connecting formation, wherein the flexible conduit is connected to the conduit connecting formation.

Preferably the breast pump comprises an adhesive member for holding the nipple engaging portion in position on the user's breast.

Preferably the adhesive member is reusable.

Preferably the adhesive member is replaceable.

Preferably the breast pump further comprises a second nipple engaging means, wherein the second nipple engaging means is connected to the inlet by the flexible conduit.

Preferably the breast pump comprises a ring configured to connect the lid to the body of the container.

According to a second embodiment of the present invention there is provided a kit of parts for a breast pump, the kit comprising:
- a deformable container body;
- a container lid comprising an inlet and a vent, wherein the vent is provided with a one way vent valve;
- a nipple engaging means for receiving milk expressed from a user's breast; and
- a flexible conduit configured to fluidly connect the nipple engaging means and the inlet of the deformable container such that when the breast pump is in use, milk expressed from the breast flows from the nipple engaging means through the conduit to the container.

Preferably, the kit comprises a ring configured to connect the container lid to the deformable container body.

Preferably, the kit comprises an adhesive member for holding the nipple engaging portion in position on the user's breast.

According to a third embodiment of the present invention there is provided a nipple shield for a breast pump comprising:
- an annular flange adapted to engage a user's breast;
- a conduit connecting portion configured to removably connect to a flexible conduit;
- a passage fluidly connecting an interior of the flange and the conduit connecting portion to allow milk expressed from the user's breast to flow into the conduit;
wherein the conduit connecting portion is configured to direct the conduit away from the nipple shield in a direction such that, in use, at least an end portion of the conduit connected to the conduit connecting portion lies substantially alongside the user's body.

Preferably, the nipple shield comprises an adhesive member for holding the nipple shield in position on the user's breast.

Preferably, the nipple shield comprises a groove configured to receive the adhesive member therein.

Preferably, the adhesive member comprises a central opening correspondingly sized to fit into the groove.

Preferably, the adhesive member is shaped and sized to adhere to both the flange and the user's breast in use.

Preferably, the nipple shield is resiliently deformable.

Preferably, the nipple shield protrudes from a surface of the user's breast by a distance of 35mm or less.

Further aspects of the invention, which should be considered in all its novel aspects, will become apparent to those skilled in the art upon reading of the following description which provides at least one example of a practical application of the invention.

### Brief Description of the Drawings

One or more embodiments of the invention will be described below by way of example only, and without intending to be limiting, with reference to the following drawings, in which:
- Figure 1: is a perspective view of a breast pump of the present invention in use;
- Figure 2: is a perspective view of the breast pump of Figure 1;
- Figure 3: is a top view of a lid of the breast pump of Figure 1;
- Figure 4: is a bottom view of a lid of the breast pump of Figure 1;
- Figure 5: is an exploded bottom perspective view of the lid of the breast pump of Figure 1;
- Figure 6: is a perspective view of the breast pump of Figure 1 configured for use with two breasts simultaneously;
- Figure 7: is a top view of a container of the breast pump of Figure 1 with section A-A indicated;
- Figure 8: is a cross section view of the container of the breast pump of Figure 1 through section A-A;
- Figure 9: is a perspective view of a nipple engaging means of the breast pump shown in Figure 1;
- Figure 10: is a cross section view through the centre of the nipple engaging means of the breast pump shown in Figure 1; and
- Figure 11: is a perspective view of an adhesive member of the breast pump of Figure 1.

### Detailed Description of Preferred Embodiments of the Invention

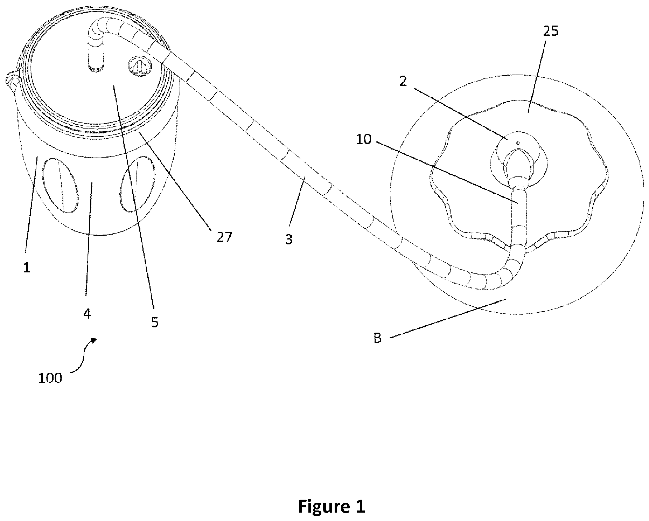

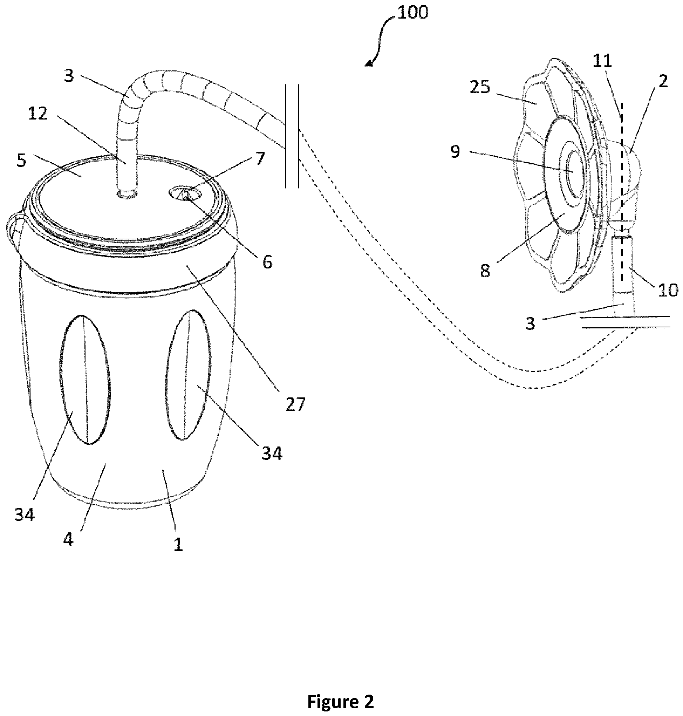

Referring first to Figures 1 and 2, a breast pump of the present invention is generally referenced by arrow 100.

The breast pump 100 comprises a container 1, at least one nipple engaging means 2, and a flexible conduit 3.

The container 1 comprises a deformable body 4 and a lid 5. The container body 4 is preferably formed from silicone, but may also be formed from another soft, flexible material, such as a rubber, thermoplastic elastomer or flexible plastic. In other embodiments (not shown), the container body 4 may comprise a non-elastic material biased into its original shape and size by a spring element. The lid 5 is preferably formed from silicone although another suitable material such as a rubber, thermoplastic elastomer or flexible plastic may be used in alternative embodiments.

The container 1 is further provided with a vent 6. In the embodiment shown the vent 6 is provided through the lid 5 of the container 1 to reduce the chance of milk accidentally being ejected from the vent 6, but in other embodiments (not shown) the vent 6 may be provided in a wall of the body of the container 1 above the expected maximum level of the milk contained in the container 1.

The vent 6 is provided with a one way valve 7 which allows air to flow out of the container 1 when the container 1 is deformed. In this way a partial vacuum is created inside the container 1 as the container 1 resiliently returns to its original shape.

Referring to Figures 1, 2 and 10, the nipple engaging means 2 comprises an annular flange 8 adapted to engage the user's nipple and breast B. In the embodiment shown the flange 8 is near planar, but is slightly angled such that it forms a conical frustrum. In one embodiment the diameter of the flange is around 50mm, and the height of the nipple engaging means (that is, the distance the body of the nipple engaging means extends from the surface of the user's breast) is around 35mm or less.

A passage 9 extends from an interior of the annular flange 8 to a conduit connecting portion 10 configured to removably connect to the conduit 3. The conduit connecting portion 10 has a central axis 11 which is substantially parallel to a plane of the annular flange 8 such that a conduit 3 connected to the conduit connecting portion 10 extends parallel to the user's body. The flange 8 is provided circumferentially about a central axis 32.

The conduit connecting portion 10 may be configured to direct the conduit 3 away from the nipple engaging means 2 in a direction such that, in use, at least an end portion of the conduit 3 connected to the conduit connection portion 10 lies substantially alongside the user's body in use. There may be a spacing between the user's body and a conduit lying alongside the user's body, but a portion of a conduit that is aligned generally parallel to the surfaces along the user's body is to be considered to be "alongside" the user's body (as opposed to extending away from the user's body in a generally perpendicular direction to the surfaces of the user's body). The central axis 11 of the conduit connection portion 10 may be aligned to extend away from the annular flange 8 in a radial direction with respect to the central axis 32 of the flange 8. In anatomical terms, conduit connecting portion 10 may be configured to direct the conduit 3 away from the nipple engaging means in an inferior, superior, lateral or medial direction. It may be more difficult for a user to wear clothing (at least discretely) over the nipple engaging means 2 if the conduit 3 is forced to extend directly away from the user's body, e.g. in an anterior direction.

In embodiments in which the annular flange 8 is frustoconical, horn-shaped, breast shaped, spherical or the like, rather than perfectly planar, the central axis 11 may be substantially parallel to a plane aligned with the interior opening defined by the annular flange 8. The central axis 11 may be substantially parallel to the surface of the user's breast that the annular flange 8 engages. The central axis 11 of the conduit connecting portion 10 may be aligned perpendicularly to the direction in which the user's nipple points in use and/or the general direction in which milk flows as it is expressed from the user's breast. The annular flange 8 may be provided about a first central axis (e.g. the central axis 32 if the annular flange 8) and the conduit connecting portion 10 may comprise a second central axis (e.g. the central axis 11 of the conduit connecting portion 10) that is perpendicular to the first central axis. More generally, the passage 9 comprises a bend 33 between the annular flange 8 and the conduit connecting portion 10. The bend 33 is substantially 90 degrees in the illustrated embodiment. In other embodiments the bend 33 may be more or less than 90 degrees, such as 45 degrees, 60 degrees, 75 degrees or 100 degrees, for example.

A conduit connecting portion 10 that connects to the conduit 3 to allow the conduit 3 to run alongside the user's body may enable the nipple engaging means 2 to be low profile, may enable the conduit 3 to be discrete and may enable the system to be less visible in use than if the conduit connection portion 10 extends directly away from the user's body.

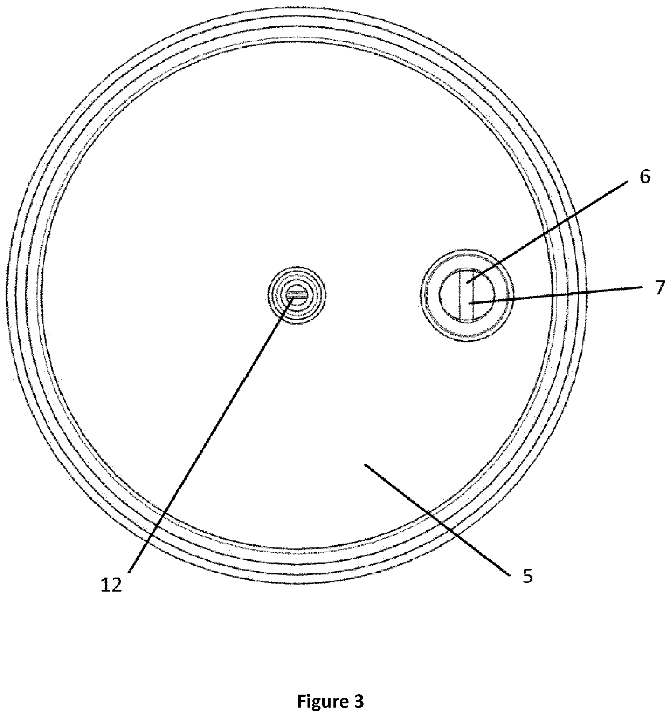

The flexible conduit 3 extends between the nipple engaging means 2 and the container 1 such that the passage 9 in the nipple engaging means 2 is in fluid communication with the interior of the container 1. The conduit 3 is connected to a conduit connecting portion 12 of the container 1. As seen in Figures 2 and 3, in preferred embodiments the conduit connecting portion 12 is provided to the lid 5 of the container 1, but in other embodiments (not shown) it may be provided to a wall of the body 4 of the container 1.

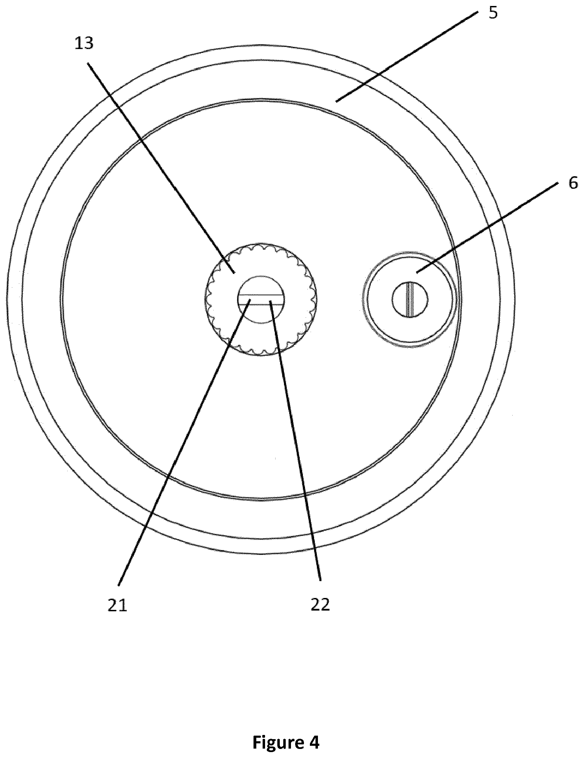

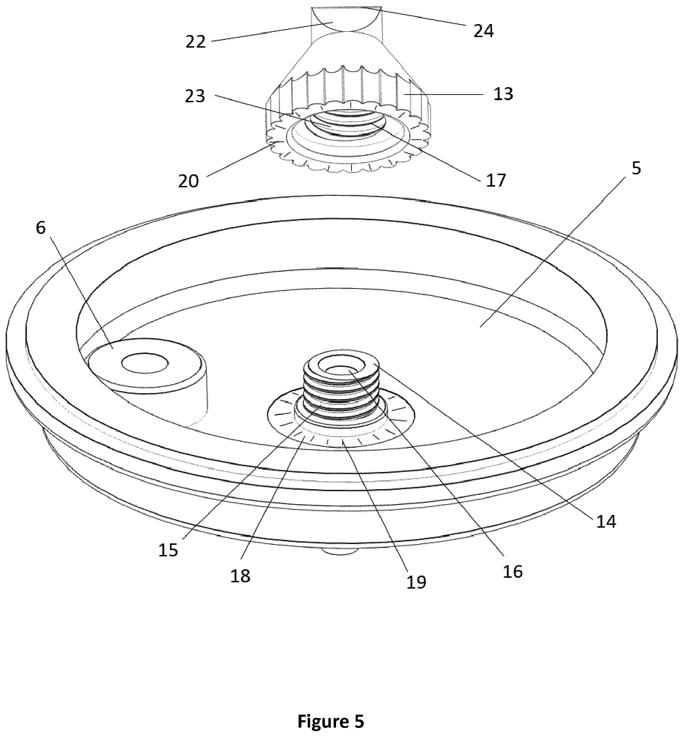

Referring next to Figures 4 and 5, in the embodiment shown a one way inlet valve 13 is connected to the lid 5 to allow milk to flow from the nipple engaging means 2 to the interior of the container 1 while preventing backflow from the container 1 to the nipple engaging means 2. In other embodiments (not shown) the inlet valve 13 may be provided in another position between the nipple engaging means 2 and the container 1 (i.e. in line with the conduit 3) or may be connected to or may be integral with the nipple engaging means 2. The container 1 comprises a plurality of windows 34, in this embodiment. The windows 34 advantageously allow the user to view the interior of the container. This enables the user to see how much milk has been collected. In this embodiment there are six windows 34. In other embodiments there may be more or fewer than six windows 34 and in some embodiments the container 1 may not comprise any windows. The windows 34 may be formed from clear silicone portions of the container 1. The windows 34 are oval in this embodiment but may be rectangular, circular or any other shape in other embodiments. In some embodiments the container 1 may comprise one or more windows extending the full height of the container.

The one way inlet valve 13 is preferably removeable from the lid 5 for cleaning. As shown in Figure 5, the lid 5 is preferably provided with a spigot 14 which is threaded on an external surface 15 thereof. A passageway 16 extends through the spigot 14 to the conduit connecting portion 12 on the opposite side of the lid 5 to the side visible shown in Figure 5. The one way inlet valve 13 is provided with an internal thread 17 to threadingly engage the spigot 14. In one embodiment the base 18 of the spigot 14 is provided with a plurality of angled teeth 19 which engage complementary angled teeth 20 provided on the base of the one-way valve 13 to form a ratchet mechanism which resists accidental detachment of the one-way valve 13 from the lid 5. The teeth 19, 20 are shaped such that the interlocking between the teeth 19, 20 can be overcome if a sufficient force is applied, such that the one way inlet valve 13 can be removed from the lid 5 intentionally by the user when required.

In the embodiment shown the one way inlet valve 13 is a "duckbill" type which has an aperture 21 formed in a ridged formation 22 (shown in Figures 4 and 5) such that a positive pressure difference between an inlet 23 and an outlet 24 of the one way inlet valve 13 (shown in Figure 5) forces the aperture 21 open, but a positive pressure difference between the outlet 24 and the inlet forces 23 the aperture 21 closed. In use, the aperture 21 may open in response to a lower pressure in the container 1 with respect to in the conduit 3, and close in response to a higher pressure in the container 1 with respect to in the conduit 3. The one way inlet valve 13 may be formed in a closed position (e.g. the ridged formation 22 may be formed such that there is substantially no aperture therebetween), so that only upon the existence of a lower pressure in container with respect to the conduit 3 will the one way inlet valve 13 open.

The one way inlet valve 13 is preferably made from silicone. Other valve types may be used, although it is preferred that the design of the valve allows for thorough cleaning of all internal surfaces and volumes. The vent valve 7 is preferably also of a duckbill type, but maybe any other suitable type.

The flexible conduit 3 connects the conduit connecting portion 10 of the nipple engaging means 2 and conduit connecting portion 12 of the container 1. The flexible conduit 3 is preferably at least 500mm long, more preferably at least 800mm long. The flexible conduit 3 in this example is a silicone tube. In other embodiments, the flexible conduit 3 may be from another suitable food grade material such as vinyl or polyethylene.

Referring again to Figure 1, in use the nipple engaging means 2 is placed over the user's nipple and breast B and is optionally held in place by an adhesive member 25, as shown in Figure 1, although (as explained below) the adhesive member may not be essential. In the embodiment shown the adhesive member 25 is approximately annular in shape and is configured to overlap the annular flange 8. The adhesive member 25 is preferably made from a nylon outer, a cotton inner and hot melt adhesive on one side to provide an adhesive surface. The adhesive member 25 is preferably reusable. The adhesive applied to the adhesive member 25 may have sufficient adhesiveness to enable the adhesive member 25 to be used multiple times. The adhesive member 25 is also replaceable and can be separated from the nipple engaging means 2 to be replaced when necessary.

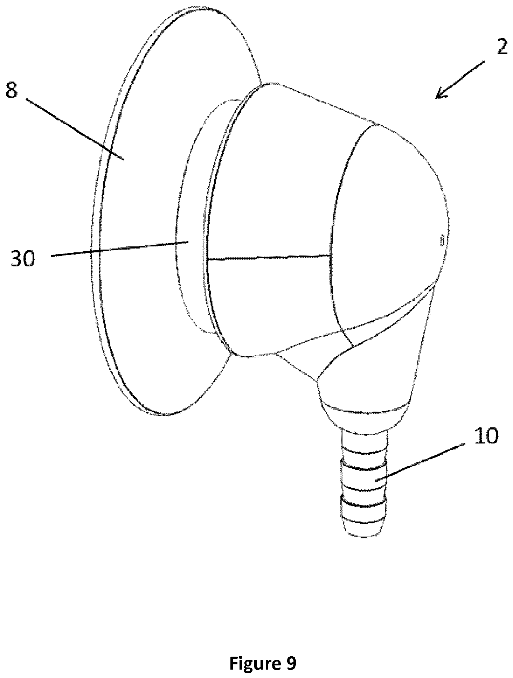

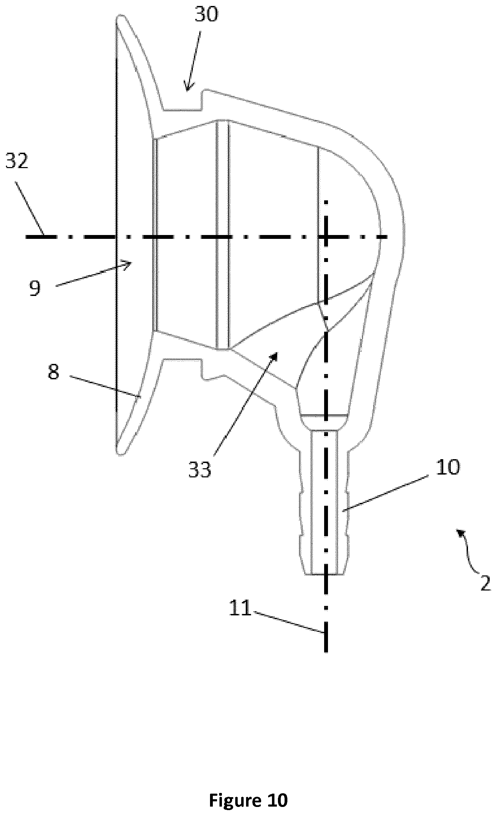

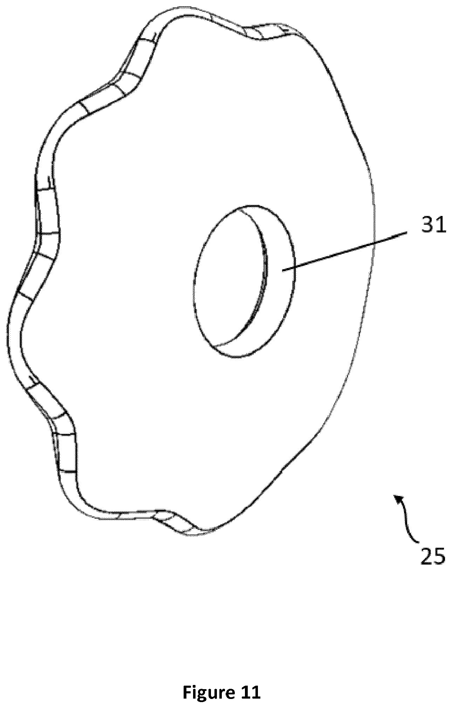

Figures 9 and 10 show the nipple engaging means 2 in isolation. In this embodiment, the nipple engaging means 2 comprises a nipple shield. As shown, the nipple engaging means 2 comprises the flange 8 and conduit connecting means 10 already described. Additionally, the nipple engaging means 2 comprises a groove 30. The groove 30 is formed in the body of the nipple engaging means proximate the flange 8. The groove 30 is an annular recess configured to receive an adhesive member for holding (e.g. temporarily adhering) the nipple engaging means 2 in position on a user's breast. Figure 11 shows the adhesive member 25 in isolation. The adhesive member 25 comprises a central opening 31. The groove 30 and central opening 31 have a corresponding shape and size to enable the periphery of the central opening 31 to fit into the groove 30, facilitating assembly of the adhesive member 25 to with the nipple engaging means 2. In the preferred embodiment, the adhesive member 25 is shaped and sized to adhere to both the flange 8 and the user's breast in use.

With the nipple engaging means 2 in place, the deformable body 4 of the container 1 is squeezed by the user, thereby venting air out from the interior of the container 1 through the vent valve 7. As the deformable body 4 returns to its original shape, a partial vacuum is created in the internal volume of the container 1. The partial vacuum is communicated to the nipple engaging means 2 by the conduit 3 and milk expressed from the nipple is sucked though the conduit 3 into the container 1. The orientation of the one way inlet valve 13 allows the milk to flow therethrough into the container 1 due to the partial vacuum.

The nipple engaging means 2 is preferably formed from a resiliently deformable material, for example, silicone, or another suitable biocompatible material such as a thermoplastic elastomer, latex or other rubber. In this way the nipple engaging means is able to deform and securely engage the nipple and/or the breast under action of the partial vacuum. Additionally, the nipple engaging means 2 is able to be squeezed before being placed over the user's breast and nipple to create its own partial vacuum to enhance its engagement to the user's nipple and breast.

The one way inlet valve 13 ensures that even when the container 1 contains milk, squeezing the container 1 does not result in milk flowing through the conduit 3 towards the nipple engaging means 2.

Because the flexible conduit 3 is at least 500mm long, the container 1 need not be held near the breast by the user when in use. The flexible conduit 3 preferably has a sufficient length to extend out from under a user's shirt and more preferably to extend further, for example to enable the container 1 to be placed on a convenient surface or in a bag or purse.

Because the nipple engaging means 2 is relatively small (for example around 50mm x 50mm x 35mm) it can be concealed beneath the user's clothes. This, coupled with the ability to position the container 1 remotely from the nipple engaging means 2, and the fact that the apparatus creates relatively little noise, means that the apparatus can be used in situations where breast pumps of the prior art could not discretely be used.

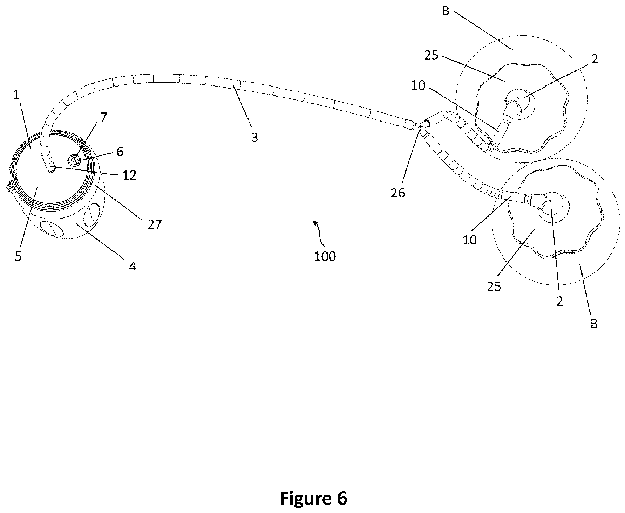

As shown in Figure 6, in some embodiments the breast pump may be provided with two nipple engaging means 2 such that the user can pump milk from both breasts B simultaneously. In a preferred embodiment the breast pump system is provided with a T or Y joiner 26 so that milk from both breasts enters the container 1 through a single inlet valve 13, and preferably though a single conduit connecting portion 12 (although in some embodiments two conduit connecting portions may be provided which feed into a single inlet valve).

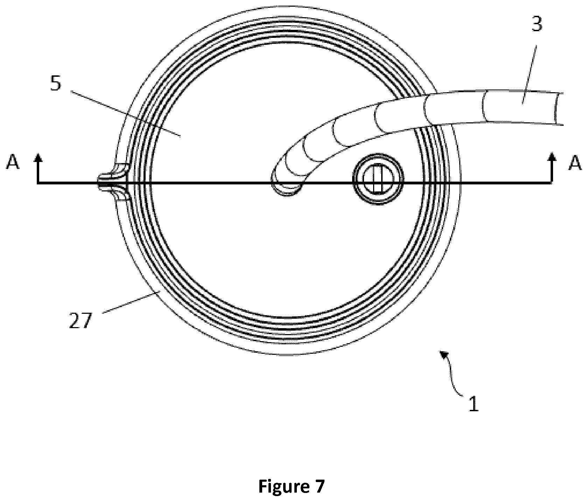

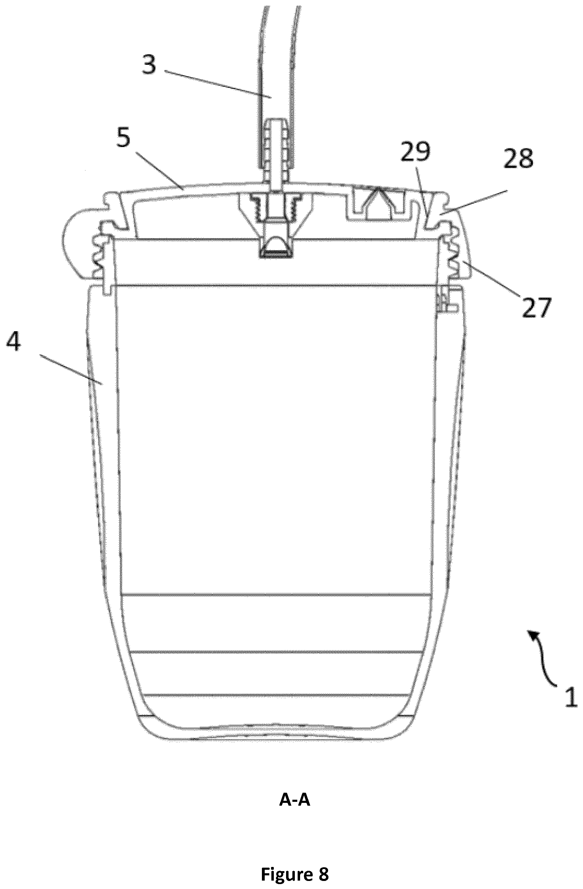

With reference to Figures 1, 2 and 6-8, in a preferred embodiment the container 1 comprises a ring 27. The ring 27 provides an attachment means by which the body 4 of the container 1 and the lid 5 are able to be removably connected. The ring 27 may be a rigid component configured to removably connect to both the body 4 of the container 1 and the lid 5 of the container. The ring is preferably formed from polypropylene although in other embodiments may be formed from PET, HDPE, polycarbonate, stainless steel, aluminium or glass as examples. The ring 27 and container 1 have the same or a similar form to the container and ring described in International (PCT) Application No. PCT/NZ2018/050171, the entire contents of which are incorporated herein by reference. The container 1 and ring 27 may connect to one another in the same ways as described in that application.

The ring 27 and the upper portion of the body 4 of the container 1 may have complimentary threaded portions enabling the ring 27 to be screwed onto the body 4 of the container 1. The body 4 of the container 1 may have one or more rigid portions to reinforce the screw thread and/or the portion of the container 1 comprising the screw thread. In some examples the upper portion of the body 4 of the container 1 may be overmoulded with a substantially rigid plastic portion comprising the screw thread. In alternative embodiments, the breast pump may comprise a ring member which connects to the body 4 of the container 1 in another manner, such as by way of a friction fit, flange-groove connection, releasable snap-fit, bayonet fitting and the like.

In the preferred embodiment the lid 5 and ring 27 are separable from one another. The lid 5 is received within the ring 27. The flexible silicone material forming the lid 5 in this embodiment is able to fit around an inwardly projecting flange 28 of the ring 27 to removably connect to the ring 27. The lid 5 may comprise a groove 29 around its periphery configured to receive the flange 28. In alternative embodiments the lid 5 may comprise a flange The lid 5 may be able to fit into place from below the ring 27 and in some embodiments may be sufficiently flexible to fit into place from above the ring 27, enabling the lid 5 to connect to the ring 27 while the ring 27 is attached to the body 4 of the container 1. The ring 27, when secured to the body 4 of the container 1, may squeeze the lid 5 against the top portion of the body 4 of the container 1, creating a seal between the body 4 of the container 1 and the lid 5.

In alternative embodiments the ring 27 may be integrally formed with the body 4 of the container 1 or the lid 5. For example, the ring 27 may be overmoulded to the upper portion of the body 4 or to the periphery of the lid 5.

In further alternative embodiments the container 1 may not comprise a ring 27. The upper portion of the body 4 of the container 1 may comprise a sufficient rigidity that the lid 5 is able to screw directly onto the body 4 of the container 1. The lid 5 may alternatively connect directly to a sufficiently rigid upper portion of the body 4 of the container 1 by a friction-fit, bayonet connection, flange-groove connection, snap fit or the like.

In preferred embodiments, the body 4 of the container and ring 27 are configured to enable the user to substitute the lid 5 with other components. In examples, the ring 27 may be configured to receive one or more of the items disclosed in International Application No. PCT/NZ2018/050171 such as the breast pump cup member, the teat, sipping straw, spoon or storage cap (identified as container lid 32 in that application).

Unless the context clearly requires otherwise, throughout the description and the claims, the words "comprise", "comprising", and the like, are to be construed in an inclusive sense as opposed to an exclusive or exhaustive sense, that is to say, in the sense of "including, but not limited to".

The entire disclosures of all applications, patents and publications cited above and below, if any, are herein incorporated by reference.

Reference to any prior art in this specification is not, and should not be taken as, an acknowledgement or any form of suggestion that that prior art forms part of the common general knowledge in the field of endeavour in any country in the world.

The invention may also be said broadly to consist in the parts, elements and features referred to or indicated in the specification of the application, individually or collectively, in any or all combinations of two or more of said parts, elements or features.

Where in the foregoing description reference has been made to integers or components having known equivalents thereof, those integers are herein incorporated as if individually set forth.

It should be noted that various changes and modifications to the presently preferred embodiments described herein will be apparent to those skilled in the art. Such changes and modifications may be made without departing from the spirit and scope of the invention and without diminishing its attendant advantages. It is therefore intended that such changes and modifications be included within the present invention.

## Claims

1. A breast pump comprising:
• a container comprising a deformable body and a lid, the container further comprising an inlet and a vent, wherein the vent is provided with a vent valve which is configured to allow air flow out of the container through the vent but not into the container through the vent;
• a nipple engaging means for receiving milk expressed from a user's breast; and
• a flexible conduit fluidly connecting the nipple engaging means and the inlet of the deformable container,
wherein, in use, milk expressed from the breast flows from the nipple engaging means through the flexible conduit to the container.

2. The breast pump of claim 1, wherein the flexible conduit is at least 500mm long.

3. The breast pump of claim 1 or claim 2, wherein the flexible conduit comprises a silicone tube.

4. The breast pump of any one of claims 1- 3, wherein the flexible conduit has an outside diameter of no more than 6mm.

5. The breast pump of any one of claims 1 - 4, wherein the conduit connecting portion is configured to direct the flexible conduit away from the nipple engaging means in a direction such that, in use, at least an end portion of the flexible conduit connected to the conduit connecting portion lies substantially alongside the user's body in use.

6. The breast pump of any one of claims 1 - 5, wherein in use, the nipple engaging means protrudes from a surface of the user's breast by a distance of 35mm or less.

7. The breast pump of any one of claims 1- 6, wherein the nipple engaging means is resiliently deformable.

8. The breast pump of any one of claims 1- 7, wherein the breast pump comprises an inlet valve between the nipple engaging means and an interior of the container, wherein the inlet valve allows fluid to flow from the nipple engaging means towards the interior of the container, but not from the interior of the container towards the nipple engaging means.

9. The breast pump of claim 8, wherein the inlet valve is connected to or integral with the container.

10. The breast pump of claim 9, wherein one or both of the inlet valve and the vent valve comprises a duckbill valve.

11. The breast pump of any one of claims 1 - 10, wherein the container comprises a conduit connecting formation, wherein the flexible conduit is connected to the conduit connecting formation in use.

12. The breast pump of any one of claims 1 - 11, wherein the breast pump comprises an adhesive member for holding the nipple engaging means in position on the user's breast.

13. The breast pump of claim 12, wherein the adhesive member is reusable and/or replaceable.

14. The breast pump of any one of claims 1 - 13, further comprising a second nipple engaging means, wherein the second nipple engaging means is connected to the inlet by the flexible conduit.

15. A kit of parts for a breast pump, the kit comprising:
• a deformable container body;
• a container lid comprising an inlet and a vent, wherein the vent is provided with a one way vent valve;
• a nipple engaging means for receiving milk expressed from a user's breast; and
• a flexible conduit configured to fluidly connect the nipple engaging means and the inlet of the deformable container such that when the breast pump is in use, milk expressed from the breast flows from the nipple engaging means through the conduit to the container.
